## Europäisches Patentamt

## European Patent Office

## Office européen des brevets

(11) Publication number: **0 214 638**
**A2**

(12) **EUROPEAN PATENT APPLICATION**

(21) Application number: **86112385.9**

(22) Date of filing: **08.09.86**

(51) Int. Cl.⁴: **C 12 N 15/00**
**C 12 N 1/20, C 12 N 1/18**
**//(C12N1/20, C12R1:19)**

(30) Priority: **09.09.85 US 774219**

(43) Date of publication of application:
**18.03.87 Bulletin 87/12**

(84) Designated Contracting States:
**DE FR GB IT**

(71) Applicant: **G.D. Searle & Co.**
**P.O. Box 1045**
**Skokie Illinois 60076(US)**

(72) Inventor: **Carter, Bruce Leonard Andrew**
**90 Ilsip Road**
**Oxford(GB)**

(72) Inventor: **Doel, Sheila May**
**Home Farm House 103 High Street**
**Chalgrove(GB)**

(72) Inventor: **Goodey, Andrew Robert**
**14 Bryanstone Avenue**
**Guildford(GB)**

(74) Representative: **Beil, Hans Christoph, Dr. et al,**
**Beil, Wolff und Beil Rechtsanwälte Adelonstrasse 58**
**D-6230 Frankfurt am Main 80(DE)**

(54) **Yeast promoters.**

(57) This invention encompasses a plasmid which can be used to express exogenous genes in yeast for the production of useful proteins. The plasmid is characterized as a 9 kilobase pair vector having origins for replication and selectivity markers for both yeast and bacteria. The plasmid has a yeast promoter segment and a unique restriction site at the 3' end of the yeast promoter for inserting a DNA segment which codes for a protein to be expressed. The plasmid is contained in *E.coli* ATCC 53229.

FIGURE 1

Our No. 25 228

G.D. Searle & Co.

P.O.Box 1045

USA – Skokie, Il. 60076

YEAST PROMOTERS

BACKGROUND OF THE INVENTION

This invention pertains to the art of producing
proteins from yeast by genetically engineering yeast with
plasmids containing a yeast promoter and DNA segment
coding for the protein to be produced.

## DESCRIPTION OF THE PRIOR ART

The prior art describes a number of yeast promoter systems: Nucleic Acid Research 10 7791 (1984) and 11 1657 (1983); J. Biol. Chem 158 2193 (1983); and Science 219 620 (1983); Nature 308 622 (1984) and Cell 30 933 (1982). Several promoters have been used to express heterologous genes on plasmids in yeast, for example, glyceraldehyde-3-phosphate dehydrogenase Urdrea et al. PNAS 80 7461 (1983); Gal 1, Goff et al. Gene 27 35 (1984); European Patent EP109560 A 2 (1984); and phosphoglycerate kinase, Mellor et al. Gene 23 215 (1985). The present invention differs in that a promoter has been directly selected from a yeast genomic library for its ability to express a heterologous gene extraordinarily well.

## BRIEF DESCRIPTION OF THE DRAWINGS

Figure 1 restriction map of plasmid SD 922.

Figure 2 restriction map of the promoter segment isolated from a yeast genomic library

Figure 3 A DNA fragment containing coding for a modified interferon and the terminator sequence from yeast alcohol dehydrogenase bounded by EcoR I and Bam H I sites.

Figure 4 A synthetic 157 base pair DNA fragment which reconstructs a portion of the 5' terminus of the modified interferon gene to the EcoR I site and link it to the promoter in the 922 plasmid.

Figure 5 A plasmid resulting from the combination of the plasmid of Figure 1 with DNA segments in Figures 3 and 4.

## DETAILED DESCRIPTION OF THE INVENTION

This invention encompasses a plasmid having a strong yeast promoter fragment, selectable markers for Leu2 and ampicillin resistance plus 2 u and pAT153 sequences to facilitate replication in yeast and _E.coli_.

The yeast promoter fragment is obtained from a genomic library of fragments obtained by Bg1II restriction of yeast DNA. The Bg1II restriction fragments are inserted into the unique Bg1II site of a plasmid containing the Herpes Simplex thymidine kinase gene, in addition to the above described markers. Only when promoter fragments are cloned in front of the thymidine kinase gene will yeast transformed with this plasmid grow in the presence of folate antagonists.

4

The plasmid having the most active promoter is selected by measurement of thymidine kinase activity in the cell extract.

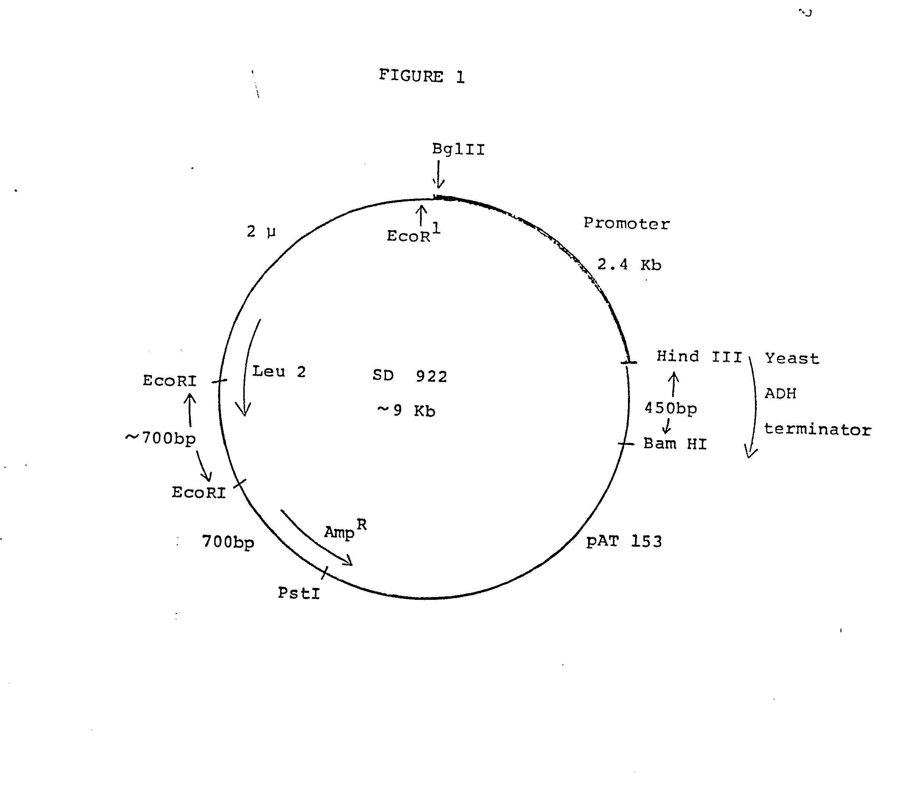

The promoter fragment is identified by DNA sequencing and modified so that there is a unique _HindIII_ restriction site in the 3' end of the yeast promoter. This modified plasmid is used to transform E. coli and the transformed E. coli is deposited as ATCC 53229 at the American Type Culture Collection, 12301 Parklawn Drive, Rockville, MD 20852 under conditions such that it is available to the public. The plasmid is a 9 Kb entity identified as SD 922. The restriction map for SD 922 is shown in Figure 1.

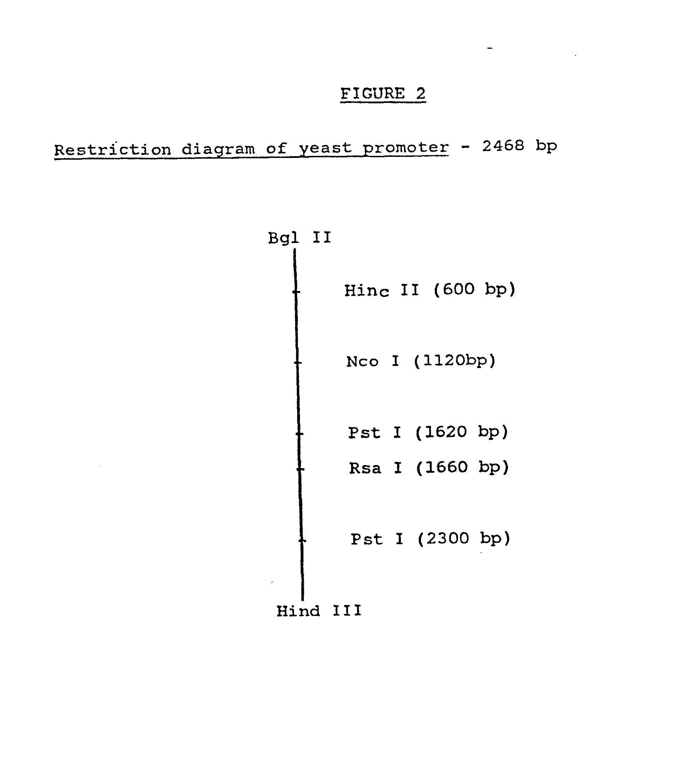

The promoter segment is contained in the clockwise Bgl II to Hind III segment of SD 922. This promoter segment between the clockwise Bgl II to Hind III sites is characterized as a 2400 base pair DNA segment having a restriction map shown in Figure 2. The restriction sites are indicated in base pairs (bp) from the Bgl II site. Thus the invention encompasses a yeast promoter segment having about 2400 base pairs, terminating with Bgl II and Hind III sites and having the following sites indicated in base pairs from the Bgl II end.

Hinc II (600 bp)

NcoI (1120 bp)

Pst I (1620 bp, 2300 bp)

Rsa I (1660 bp)

A DNA segment which codes for a protein desired to be
produced and which has termini compatible for insertion
into the Hind III site is inserted into SD 922.  The
transformed yeast are cultured and the desired protein is
isolated by conventional techniques.

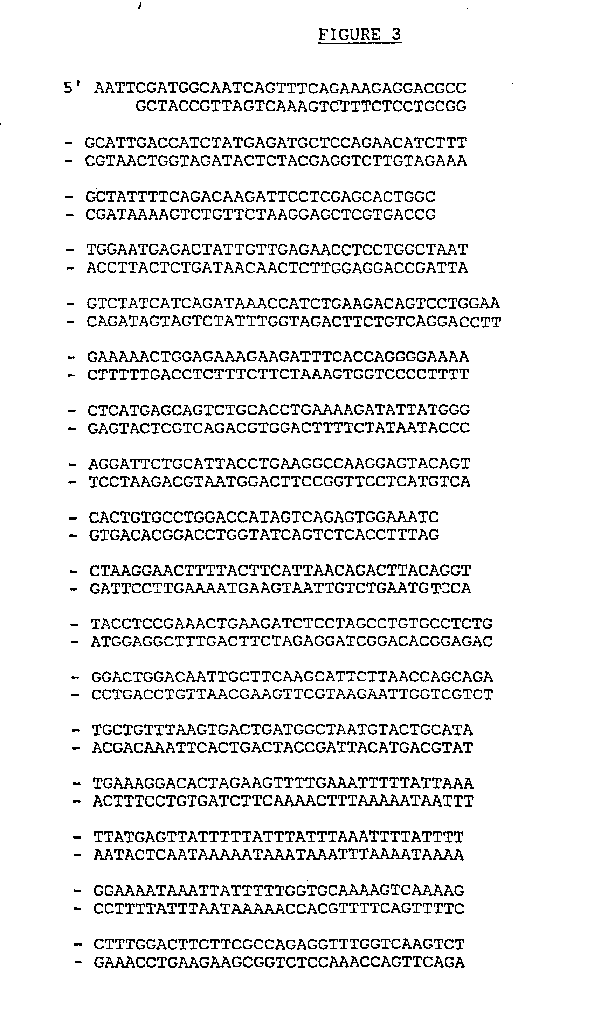

Yeast strain SLP 1B transformed with SD 922 containing DNA
of Figure 3 coding for hybrid interferon grew much more
quickly than the equivalent construct containing pyruvate
kinase promoter - 3 days versus 10 days respectively.
Also after 2 days non-selective growth in liquid culture
and plating out to single cells non-selectively, the
majority of colonies still grew strongly on  minus leu-plates
whereas the equivalent construct with the pyruvate kinase
promoter gave nearly 100% poor growers.

Those skilled in the genetic engineering arts will
recognize a large number of techniques for modifying DNA
segments which code for a wide variety of proteins for
insertion into a Hind III site.

The hereinafter set forth examples illustrate the
invention and are not intended to limit it in spirit or
scope.

### Example 1

Construction of a plasmid for expressing a modified
interferon in yeast.

1 µg of plasmid DNA SD 922 obtained from E. coli ATCC 53229 was restricted in a volume of 20 µl of core buffer containing 5 units of Hind III restriction enzyme. The restriction was carried out for 1 hour at 37°C after which time the solution was made 150 millimolar NaCl and 5 units of Bam H I restriction enzyme were added. The reaction was incubated for a further hour at 37°C. 5µl of loading buffer was added and the mixture was separated by electrophoresis on a 1% agarose gel. The major band was excised and electroeluted. The final pellet was dissolved in 20 µl of water.

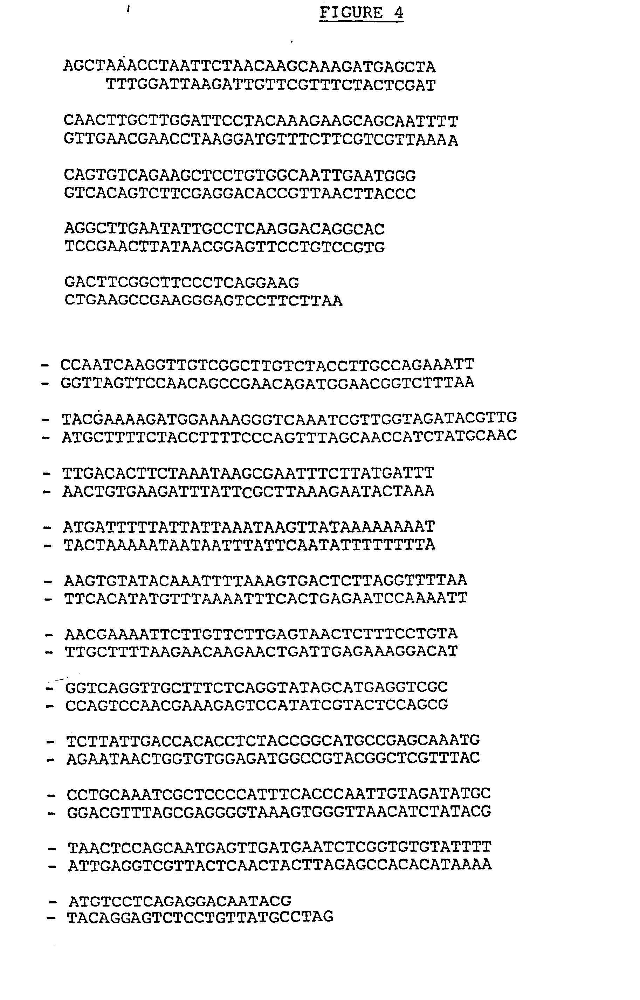

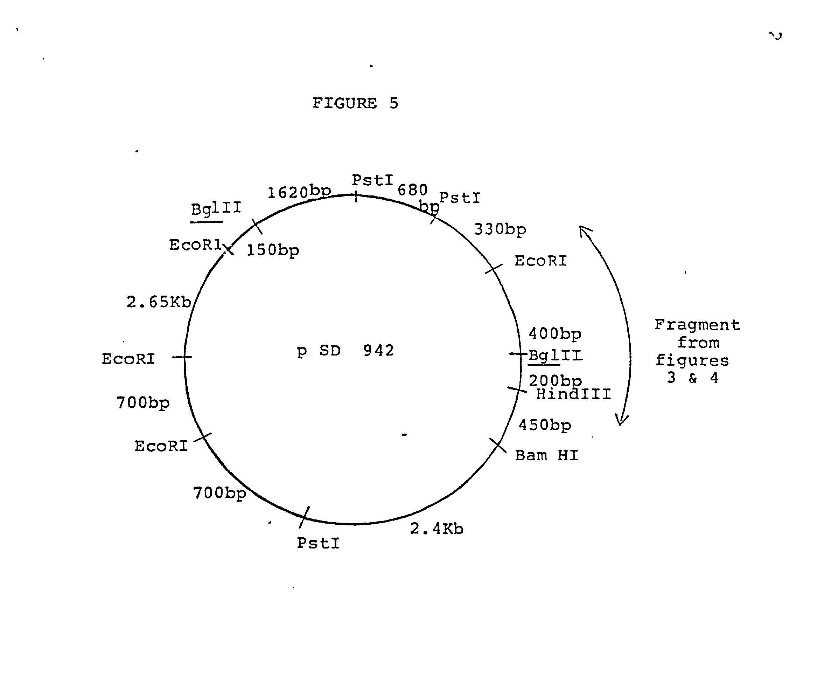

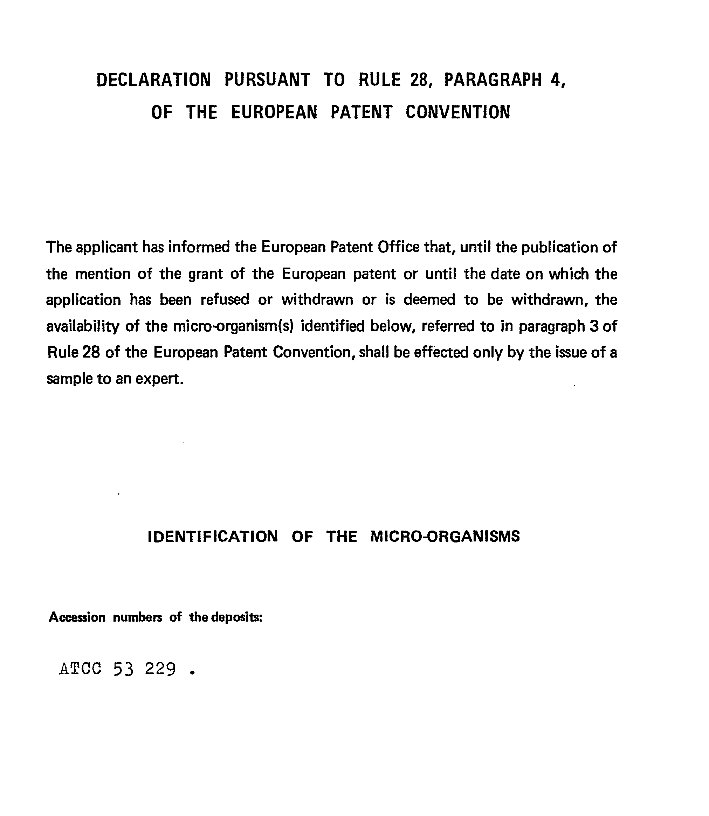

The ligation is performed by standard methods in 20 µl of buffer containing 15 ng of the DNA fragment of EcoRI/Bam HI fragment; 15 ng of the DNA segment of Figure 3 and 2 ng of the DNA segment of Figure 4. The plasmid is isolated and restriction sites identified by standard techniques. The plasmid identified as SD 942 in figure 5 was isolated. The DNA sequence in Figure 3 codes for a modified interferon.

Yeast transformation

Sphaeroplasts from strain SLP1B of Saccharomyces cerevisiae were made by standard methods and 2 ug SD 942 was added to a 50 ul aliquot. After transformation the sphaeroplasts were embedded in minimal minus leucine media containing 1 M sorbitol in agar plates and incubated at 30°C for 4 days. The transformants were streaked onto minimal media minus leu/sorbitol plates and incubated at 30° C for 4 days.*

## Expression of Modified Interferon

10 ml cultures of YEPD (1% yeast extract, 2% peptone, 2% glucose) were inoculated with cells from the streaks of SD 942 and grown for 20 hours, 44 hours and 4 days with shaking at 30°.

The cells were counted on the Coulter counter, harvested by centrifugation (4K, 5') and lysed in 1 ml PBS/0.1% BSA, using glass beads. The supernatant was extracted, diluted 1/1000 with PBS/0.1% BSA and assayed for IFN activity. The activities were compared to the equivalent construct using the PK promoter (pAYE 115).

| Day | SD 942 | pAYE 115 (PK promoter) |
|---|---|---|
| 1 | $2 \times 10^8$ u/$\ell$/$10^{11}$ cells | $1.4 \times 10^8$ u/$\ell$/$10^{11}$ cells |
| 2 | $8 \times 10^7$ u/$\ell$/$10^{11}$ cells | $1.4 \times 10^7$ u/$\ell$/$10^{11}$ cells |
| 4 | $2 \times 10^7$ u/$\ell$/$10^{11}$ cells | $0.5 \times 10^7$ u/$\ell$/$10^{11}$ cells |

Stationary phase had been reached by day 2.

---

*The transformants were streaked onto minimal media, minus leu/
sorbitol plates and incubated at 30°C for 2 days and
stored at +4°C.

A stationary overnight culture of SD 942 and pAYE 115 was grown in YEPD and 50 ul of each inoculated into 10 ml flasks of YEPD. These were shaken for 24 hours at 30° and 50 ul was transferred to fresh 10 ml of medium. The remaining cells were counted, collected by centrifugation, lysed and assayed as described above. This process was repeated for 9 days.

The IFN titre of SD 942 dropped from $6 \times 10^8$ units/ℓ/$10^{11}$ cells to $3 \times 10^8$ units/ℓ/$10^{11}$ cells over this period whereas the titre of pAYE 115 dropped from $4 \times 10^8$ units/ℓ/$10^{11}$ cells to $1 \times 10^6$ units/ℓ/$10^{11}$ cells.

What is claimed is:

1.  A plasmid SD 922 substantially as shown in figure 1.

2.  A plasmid, according to claim 1, further containing
    a DNA segment coding for a protein to be expressed
    where the DNA segment is inserted at the Hind III
    restriction site.

3.  A yeast promoter segment contained within the
    clockwise Bgl II to Hind III segment of the SD 922
    plasmid characterized by restriction sites located
    from the Bgl II site:

        Hinc II 600 bp

        NcoI 1120 bp

        Pst I 1620 bp and 2300 bp and

        Rsa I 1660 bp.

4.  E.coli ATCC 53229.

5.  Yeast containing plasmid SD 922 which has a DNA
    segment coding for a protein to be expressed inserted
    at the Hind III restriction site.

6.  A method for the production of proteins which involves
    the expression of an exogenous gene sequence by means
    of the yeast promoter segment of claim 3 included in
    a yeast shuttle vector which can be used to transform
    yeast.

FIGURE 1

## FIGURE 2

Restriction diagram of yeast promoter - 2468 bp

```
Bgl II
  │
  ┼    Hinc II (600 bp)
  │
  │
  ┼    Nco I (1120bp)
  │
  │
  ┼    Pst I (1620 bp)
  ┼    Rsa I (1660 bp)
  │
  │
  ┼    Pst I (2300 bp)
  │
Hind III
```

FIGURE 3

```
5' AATTCGATGGCAATCAGTTTCAGAAAGAGGACGCC
      GCTACCGTTAGTCAAAGTCTTTCTCCTGCGG

-  GCATTGACCATCTATGAGATGCTCCAGAACATCTTT
-  CGTAACTGGTAGATACTCTACGAGGTCTTGTAGAAA

-  GCTATTTTCAGACAAGATTCCTCGAGCACTGGC
-  CGATAAAAGTCTGTTCTAAGGAGCTCGTGACCG

-  TGGAATGAGACTATTGTTGAGAACCTCCTGGCTAAT
-  ACCTTACTCTGATAACAACTCTTGGAGGACCGATTA

-  GTCTATCATCAGATAAACCATCTGAAGACAGTCCTGGAA
-  CAGATAGTAGTCTATTTGGTAGACTTCTGTCAGGACCTT

-  GAAAAACTGGAGAAAGAAGATTTCACCAGGGGAAAA
-  CTTTTTGACCTCTTTCTTCTAAAGTGGTCCCCTTTT

-  CTCATGAGCAGTCTGCACCTGAAAAGATATTATGGG
-  GAGTACTCGTCAGACGTGGACTTTTCTATAATACCC

-  AGGATTCTGCATTACCTGAAGGCCAAGGAGTACAGT
-  TCCTAAGACGTAATGGACTTCCGGTTCCTCATGTCA

-  CACTGTGCCTGGACCATAGTCAGAGTGGAAATC
-  GTGACACGGACCTGGTATCAGTCTCACCTTTAG

-  CTAAGGAACTTTTACTTCATTAACAGACTTACAGGT
-  GATTCCTTGAAAATGAAGTAATTGTCTGAATGTCCA

-  TACCTCCGAAACTGAAGATCTCCTAGCCTGTGCCTCTG
-  ATGGAGGCTTTGACTTCTAGAGGATCGGACACGGAGAC

-  GGACTGGACAATTGCTTCAAGCATTCTTAACCAGCAGA
-  CCTGACCTGTTAACGAAGTTCGTAAGAATTGGTCGTCT

-  TGCTGTTTAAGTGACTGATGGCTAATGTACTGCATA
-  ACGACAAATTCACTGACTACCGATTACATGACGTAT

-  TGAAAGGACACTAGAAGTTTTGAAATTTTTATTAAA
-  ACTTTCCTGTGATCTTCAAAACTTTAAAAATAATTT

-  TTATGAGTTATTTTTATTTATTTAAATTTTATTTT
-  AATACTCAATAAAAATAAATAAATTTAAAATAAAA

-  GGAAAATAAATTATTTTTGGTGCAAAAGTCAAAAG
-  CCTTTTATTTAATAAAAACCACGTTTTCAGTTTTC

-  CTTTGGACTTCTTCGCCAGAGGTTTGGTCAAGTCT
-  GAAACCTGAAGAAGCGGTCTCCAAACCAGTTCAGA
```

## FIGURE 4

AGCTAAACCTAATTCTAACAAGCAAAGATGAGCTA
　TTTGGATTAAGATTGTTCGTTTCTACTCGAT

CAACTTGCTTGGATTCCTACAAAGAAGCAGCAATTTT
GTTGAACGAACCTAAGGATGTTTCTTCGTCGTTAAAA

CAGTGTCAGAAGCTCCTGTGGCAATTGAATGGG
GTCACAGTCTTCGAGGACACCGTTAACTTACCC

AGGCTTGAATATTGCCTCAAGGACAGGCAC
TCCGAACTTATAACGGAGTTCCTGTCCGTG

GACTTCGGCTTCCCTCAGGAAG
CTGAAGCCGAAGGGAGTCCTTCTTAA


- CCAATCAAGGTTGTCGGCTTGTCTACCTTGCCAGAAATT
- GGTTAGTTCCAACAGCCGAACAGATGGAACGGTCTTTAA

- TACGAAAAGATGGAAAAGGGTCAAATCGTTGGTAGATACGTTG
- ATGCTTTTCTACCTTTTCCCAGTTTAGCAACCATCTATGCAAC

- TTGACACTTCTAAATAAGCGAATTTCTTATGATTT
- AACTGTGAAGATTTATTCGCTTAAAGAATACTAAA

- ATGATTTTTATTATTAAATAAGTTATAAAAAAAAT
- TACTAAAAATAATAATTTATTCAATATTTTTTTTA

- AAGTGTATACAAATTTTAAAGTGACTCTTAGGTTTTAA
- TTCACATATGTTTAAAATTTCACTGAGAATCCAAAATT

- AACGAAAATTCTTGTTCTTGAGTAACTCTTTCCTGTA
- TTGCTTTTAAGAACAAGAACTGATTGAGAAAGGACAT

- GGTCAGGTTGCTTTCTCAGGTATAGCATGAGGTCGC
- CCAGTCCAACGAAAGAGTCCATATCGTACTCCAGCG

- TCTTATTGACCACACCTCTACCGGCATGCCGAGCAAATG
- AGAATAACTGGTGTGGAGATGGCCGTACGGCTCGTTTAC

- CCTGCAAATCGCTCCCCATTTCACCCAATTGTAGATATGC
- GGACGTTTAGCGAGGGGTAAAGTGGGTTAACATCTATACG

- TAACTCCAGCAATGAGTTGATGAATCTCGGTGTGTATTTT
- ATTGAGGTCGTTACTCAACTACTTAGAGCCACACATAAAA

- ATGTCCTCAGAGGACAATACG
- TACAGGAGTCTCCTGTTATGCCTAG

## FIGURE 5

BglII
1620bp · PstI 680 · PstI
bp
EcoR1 · 150bp
330bp
2.65Kb
EcoRI
p SD 942
400bp
BglII
200bp
EcoRI
700bp
HindIII
450bp
EcoRI
700bp
Bam HI
2.4Kb
PstI

Fragment
from
figures
3 & 4

5/5

0214638

# DECLARATION PURSUANT TO RULE 28, PARAGRAPH 4, OF THE EUROPEAN PATENT CONVENTION

The applicant has informed the European Patent Office that, until the publication of the mention of the grant of the European patent or until the date on which the application has been refused or withdrawn or is deemed to be withdrawn, the availability of the micro-organism(s) identified below, referred to in paragraph 3 of Rule 28 of the European Patent Convention, shall be effected only by the issue of a sample to an expert.

## IDENTIFICATION OF THE MICRO-ORGANISMS

**Accession numbers of the deposits:**

ATCC 53 229 .